# EUROPÄISCHE PATENTSCHRIFT

(11) **EP 2 236 949 B1**
(45) Veröffentlichungstag und Bekanntmachung des Hinweises auf die Patenterteilung: **26.06.2013**
(21) Anmeldenummer: 10155925.0
(22) Anmeldetag: 09.03.2010
(51) Int. Cl.: F24F 3/16, A61L 9/015, A61L 2/20, A61L 2/24

(54) **Dekontamination eines Raumes mittels eines benutzerdefinierten Dekontaminationsprozesses**
Decontamination of a room using a user-defined decontamination process
Décontamination d'une pièce à l'aide d'un procédé de décontamination défini par l'utilisateur

(30) Priorität: 10.03.2009 DE 102009012414
(43) Veröffentlichungstag der Anmeldung: 06.10.2010
(73) Patentinhaber: Ortner Cleanroom Engineering GmbH, 9500 Villach (AT)
(72) Erfinder: Ortner, Josef, 9872 Millstatt (AT)
(74) Vertreter: Dilg, Haeusler, Schindelmann Patentanwaltsgesellschaft mbH

(56) Entgegenhaltungen:
- WO-A2-2006/046993
- US-A1- 2006 008 379

## Beschreibung

Die vorliegende Erfindung betrifft das technische Gebiet der Dekontamination von Räumen. Die vorliegende Erfindung betrifft insbesondere eine Zentraleinheit für ein Dekontaminationssystem zum chemischen Dekontaminieren von Räumen, ein Dekontaminationssystem mit einer Zentraleinheit sowie ein Verfahren zum chemischen Dekontaminieren von Räumen, welches Verfahren mit einem Dekontaminationssystem des genannten Typs durchführbar ist.

Räume, Kammern, Geräte und Anlagen werden in der Regel mit verschiedenen Chemikalien wie beispielsweise Formaldehyd, Ethylenoxid, Minncare, Ammoniak etc. dekontaminiert. Unter dem Begriff Dekontamination sind in diesem Zusammenhang die Entfernung und/oder die Unschädlichmachung von Verunreinigungen an Personen, Objekten oder Flächen oder in beliebigen Volumina bzw. Räumen zu verstehen. Die Verunreinigungen, welche auch als Kontaminationen bezeichnet werden, können physikalischer, chemischer und/oder biologischer Natur sein. Unter dem Begriff Dekontamination soll im Rahmen dieses Dokuments insbesondere auch eine Desinfektion und/oder eine Sterilisation verstanden werden. Das Einbringen der betreffenden Chemikalie(n) in einen zu dekontaminierenden Raum kann unter Verwendung verschiedener Verfahren wie beispielsweise Vernebeln, Besprühen, Verdampfen etc. erfolgen.

Wasserstoffperoxid (H2O2) ist seit vielen Jahren und in unterschiedlichsten Branchen ein bewährtes und zunehmend beliebtes Dekontaminationsmittel. Dies gilt vor allem deshalb, weil H2O2 umweltfreundlich abbaubar ist und im Vergleich zu anderen chemischen Dekontaminationsmitteln weit weniger aufwendige Sicherheitsmaßnahmen erfordert. Zudem ist eine sehr große Bandbreite von zu dekontaminierenden Materialien gegen H2O2 beständig. H2O2 kann in flüssiger oder gasförmiger Form angewendet werden. Der Aggregatszustand und/oder die Konzentration des applizierten H2O2 kann an die jeweiligen Anforderungen angepasst werden.

Für eine Raum- oder Kammerdekontamination wird heutzutage ausschließlich die Begasung mit verdampften H2O2 verwendet. Dazu gibt es eigene Generatoren, welche H2O2 mit einer Temperatur von ca. 70°C bis 90°C und einer begrenzten Luftmenge von ca. 30 bis 60 Kubikmeter pro Stunde (m3/h) erzeugen. Die von verschiedenen Generatoren verwendete Prozesse sind jedoch von Hersteller zu Hersteller unterschiedlich und können je nach Anwendungssituation nicht oder nur bedingt eingesetzt werden.

In der Regel erfolgt die Einbringung von H2O2 in den zu dekontaminierenden Raum direkt über den Generator. Die Verteilung des H2O2 im Raum wird über freistehende, mobile Standventilatoren oder über das zentrale Lüftungssystem bewirkt. Dabei wird häufig eine bewusste Verwirbelung verwendet. Die in dem zu dekontaminierenden Raum befindliche Luft kann auch mit der Zeit mit H2O2 gesättigt werden. Dies kann jedoch zu einer unerwünschten Bildung von Kondensat und/oder zu einer Ausbildung von sog. Totzonen führen, in denen das H2O2 nicht oder nicht in ausreichender Menge eindringt.

Ein weiterer Nachteil von bekannten Dekontaminationsverfahren auf der Basis von H2O2 besteht darin, dass ein H2O2 Generator typischerweise jeweils nur für ein bestimmtes Dekontaminationssystem einsetzbar bzw. konzipiert ist, so dass die Flexibilität eines H2O2 Generators hinsichtlich verschiedener Anwendungen entsprechend eingeschränkt ist.

WO 2006/046993 A2 beschreibt ein Dekontaminationssystem zum Dekontaminieren eines Raumes auf der Basis von Wasserstoffperoxid. Das Dekontaminationssystem weist eine Zentraleinheit auf, welche einen Eingang und einen Ausgang aufweist, so dass das Dekontaminationssystem mit einem geschlossenen Kreislauf betrieben werden kann. Unter Verwendung einer Pumpe wird Raumluft von dem Eingang angesaugt und über eine Rohrleitung zu dem Ausgang befördert. In der Rohrleitung sind verschiedene Konditionier-Komponenten angeordnet.

US 2006/008379 A1 beschreibt ein Raumdekontaminationssystem auf der Basis von Wasserstoffperoxid-Dampf. Das System weist eine mittels eines Wagens verfahrbare Zentraleinheit auf, welche einen Eingang und einen Ausgang aufweist, so dass das System mit einem geschlossenen Kreislauf betrieben werden kann. Unter Verwendung einer Pumpe wird Raumluft von dem Eingang angesaugt und über eine Rohrleitung zu dem Ausgang befördert. In der Rohrleitung sind verschiedene Konditionier-Komponenten angeordnet. Sämtliche Konditionier-Komponenten sind mit einer zentralen Steuerungseinheit gekoppelt und können von dieser gesteuert werden. Der Dekontaminationsprozess kann automatisch gesteuert werden.

Der vorliegenden Erfindung liegt die Aufgabe zugrunde, eine flexibel einsetzbare Zentraleinheit für ein Dekontaminationssystem sowie ein entsprechendes Verfahren zum chemischen Dekontaminieren von Räumen zu schaffen, welche für eine Vielzahl von verschiedenen Dekontaminationstechnologien und/oder Chemikalien geeignet sind.

Diese Aufgabe wird gelöst durch die Gegenstände der unabhängigen Patentansprüche. Vorteilhafte Ausführungsformen der vorliegenden Erfindung sind in den abhängigen Ansprüchen beschrieben.

Gemäß einem ersten Aspekt der Erfindung wird eine Zentraleinheit für ein Dekontaminationssystem zum chemischen Dekontaminieren von Räumen beschrieben. Die beschriebene Zentraleinheit weist auf (a) einen Anschluss zum Anschließen einer Abgabeeinheit, welche zum Abgeben eines chemischen Dekontaminationsmittels in einen zu dekontaminierenden Raum eingerichtet ist, (b) eine Steuerungseinheit zum Steuern einer Zufuhr des chemischen Dekontaminationsmittels zu der Abgabeeinheit, und (c) eine mit der Steuerungseinheit gekoppelte Benutzerschnittstelle zum Eingeben von zumindest einer Information an die Steuerungseinheit. Dabei ist die Information mit einem benutzerdefinierten Dekontaminationsprozess verknüpft und die Steuerungseinheit ist derart eingerichtet, dass basierend auf der Information der entsprechende benutzerdefinierte Dekontaminationsprozess von der Zentraleinheit durchführbar ist. Die beschriebene Zentraleinheit weist ferner (d) eine Fluidleitung auf, welche ausgangsseitig mit dem Anschluss gekoppelt ist und welche derart eingerichtet ist, dass ein Fluid, welches das chemische Dekontaminationsmittel aufweist, zu dem Anschluss transferierbar ist. Die Fluidleitung weist auf entweder (e1) eine Unterbrechung und durch die Unterbrechung erzeugte Enden, an welchen eine in Bezug zu der Zentraleinheit externe Generatoreinheit zum Erzeugen des chemischen Dekontaminationsmittels anschließbar ist, oder (e2) einen Teilabschnitt, zu dem eine in Bezug zu der Zentraleinheit externe Generatoreinheit zum Erzeugen des chemischen Dekontaminationsmittels parallel schaltbar ist.

Der beschriebenen Zentraleinheit liegt die Erkenntnis zugrunde, dass ein Dekontaminationsprozess, welcher von der Abgabeeinheit in dem zu dekontaminierenden Raum ausgeführt wird, von der Steuereinheit der Zentraleinheit entsprechend einer benutzerdefinierten Anweisung durchgeführt werden kann. Dabei kann die Abgabeeinheit räumlich getrennt von der Zentraleinheit angeordnet sein.

Im Rahmen des benutzerdefinierten Dekontaminationsprozesses kann eine bestimmte Menge an Dekontaminationsmittel innerhalb einer bestimmten Zeitspanne an den zu dekontaminierenden Raum abgegeben werden. Dabei kann von einer Bedienperson auch ein zeitliches Profil bezüglich der Abgabemenge und/oder der Konzentration an abzugebenden Dekontaminationsmittel vorgegeben werden. Ferner kann die Abgabe des Dekontaminationsmittels auch durch eine an die jeweiligen Anforderungen angepasste Konditionierung eines Abgabefluids optimiert werden, wobei das Abgabefluid das chemische Dekontaminationsmittel zusammen mit einer gasförmigen und/oder flüssigen Trägersubstanz umfasst. Auch das chemische Dekontaminationsmittel kann in einer flüssigen und/oder in einer gasförmigen Phase vorliegen.

Die Steuerungseinheit kann derart eingerichtet sein, dass der benutzerdefinierte Dekontaminationsprozess in reproduzierbarer Weise durchgeführt wird. Dies kann bedeuten, dass der Dekontaminationsprozess auch ein bestimmter Dekontaminationszyklus sein kann, welcher in bestimmten Zeitabständen wiederholt werden kann und welcher somit dazu betragen kann, dass der zu dekontaminierende Raum zumindest zu bestimmten Zeitpunkten in einem Zustand ist, in dem chemische und/oder biologische Verunreinigungen lediglich unterhalb einer akzeptablen Konzentration vorhanden sind.

Die Steuerungseinheit kann einen Speicher aufweisen, in dem mittels EDV und/oder CAD entwickelte Simulationen für einen Dekontaminationsprozess, insbesondere einen Begasungsprozess und/oder eine Spülung des zu kontaminierenden Raumes, gespeichert werden können. Bei Bedarf kann dann ein entsprechender realer Dekontaminationsprozess durchgeführt werden.

Die Steuerungseinheit kann ferner derart eingerichtet sein, dass validierte Prozesse bzw. Dekontaminationsprofile entwickelt und durch eine geeignete Aktivierung von Komponenten der Zentraleinheit abgefahren werden können. Dabei kann das oben genannte Abgabefluid, welches zumindest das chemische Dekontaminationsmittel in flüssiger und/oder gasförmiger Form aufweist, durch zumindest eine aktivierte Komponente in geeigneter Weise konditioniert werden. Abhängig von den jeweiligen Bedürfnissen kann das Abgabefluid beispielsweise erwärmt, abgekühlt, befeuchtet, getrocknet, katalysiert, ionisiert, UV bestrahlt und/oder mit Über- oder Unterdruck beaufschlagt werden. Ferner kann der Anteil an dem Dekontaminationsmittel in dem Abgabefluid durch ein Konzentrieren oder ein Verdünnen erhöht bzw. erniedrigt werden. Somit können mit der Zentraleinheit in Hinblick auf verschiedenste Anwendungsfälle geeignete Abgabefluidkonditionen geschaffen werden.

Der zu dekontaminierende Raum kann ein beliebiger Raum wie beispielsweise ein Reinraum sein, bei dem nach einer geeigneten Dekontamination die Konzentration an chemischen und/oder biologischen Verunreinigungen zumindest unterhalb einer bestimmten Schwelle liegen soll. Im Bereich der Medizin kann der zu dekontaminierende Raum beispielsweise ein Operationsraum sein, in welchem zur Vermeidung von Infektionen bei einer zu operierenden Person eine geringe Konzentration an Keimen erforderlich ist. Das gleiche gilt im Bereich der Lebensmitteltechnik für Räume, in denen Lebensmittel aufbewahrt und/oder verarbeitet werden. Der zu dekontaminierende Raum kann auch ein Reinraum sein, in welchem im Bereich der Elektronikfertigung Halbleiterbauelemente hergestellt und/oder bearbeitet werden. Ferner kann es sich bei dem zu dekontaminierenden Raum um eine sog. Schleuse handeln, in der ein Gegenstand eingebracht und nach einer geeigneten Dekontamination wieder entnommen und insbesondere an eine weiteren Raum übergeben wird. Der zu dekontaminierende Raum kann ferner beispielsweise ein Labor oder ein bestimmter Bereich eines Labors sein. Der zu dekontaminierende Raum kann beispielsweise für eine Tierhaltung und/oder zum Durchführen von Tierversuchen vorgesehen sein. Außerdem kann der zu dekontaminierende Raum auch eine Präparationskammer sein, in der ein beliebiger Gegenstand innerhalb von der äußeren Umgebung abgetrennt präpariert werden kann, wobei unter dem Begriff "Präparation" eine beliebige Modifikation und/oder Herstellung des Gegenstandes verstanden werden kann. Die Präparationskammer kann insbesondere ein zumindest teilweise durchsichtiger Kasten sein, in dem zwei mit jeweils einem Handschuh verschlossene Eingriffsöffnungen vorhanden sind, über welche ein Benutzer den jeweiligen Gegenstand in geeigneter Weise handhaben kann. Eine solche Präparationskammer wird häufig auch als Isolator bezeichnet.

Die beschriebene Zentraleinheit ist somit vielseitig, variabel und gestaltbar für verschiedenste Dekontaminationsprozesse einsetzbar. Insbesondere kann die beschriebene Zentraleinheit für die Begasung von allen möglichen Arten von Räumen oder Volumina wie beispielsweise Isolatoren, Materialschleusen, Durchreichen, Personenschleusen, Kühlzellen, Operationsräumen, Krankenzimmer, Laborräumen, Digestorien wie beispielsweise Laborabzüge oder Apothekenabzüge und dergleichen eingesetzt werden. Ferner kann die beschriebene Zentraleinheit zur Versorgung und Steuerung von mobilen oder stationären Begasungsstationen, zur seriellen oder parallelen Versorgung und Steuerung von kombinierten Begasungssystemen wie z.B. Isolator und Materialschleuse oder zur seriellen oder parallelen Versorgung und Steuerung von Begasungssystemen für mehrere Räume verwendet werden.

Es wird darauf hingewiesen, dass die hier aufgeführten Anwendungen lediglich beispielhaft sind und dass die beschriebene Zentraleinheit zusammen mit einer angeschlossenen Abgabeeinheit auch noch für andere Dekontaminationsanwendungen verwendet werden kann.

Das chemische Dekontaminationsmittel kann von der beschriebenen Zentraleinheit an die Abgabeeinheit transferiert werden. In diesem Fall kann der Anschluss insbesondere ein Medienanschluss sein, welcher einen Transfer von Dekontaminationsmittel in flüssiger, gasförmiger und/oder in fester Form erlaubt. Das chemische Dekontaminationsmittel kann jedoch auch von einer beliebigen anderen Komponente des Dekontaminationssystems an die Abgabeeinheit übergeben werden. In diesem Fall kann eine entsprechende einen Materialfluss erlaubende Leitung zwischen dieser anderen Komponente, beispielsweise einen Generator zum Erzeugen des Dekontaminationsmittels, und der Abgabeeinheit vorhanden sein. Der an der Zentraleinheit vorgesehene Anschluss kann dann auch einfach ein Anschluss für eine rückkopplungsfreie Steuerleitung oder eine rückkopplungsbehaftete Regeleitung sein.

Das Steuern kann ein rückkopplungsfreies Steuern oder ein rückkopplungsbehaftetes Regeln sein. Im Falle einer rückkopplungsbehafteten Regelung sind selbstverständlich noch geeignete Sensoren erforderlich, welche die physikalischen und/oder chemischen Bedingungen in dem zu dekontaminierenden Raum erfassen und die entsprechenden Messwerte über eine kabelgebundene oder eine drahtlose Verbindung direkt oder indirekt an die Steuereinheit übermitteln. Eine physikalische Raumbedingung kann beispielsweise die Temperatur und/oder die Luftfeuchtigkeit sein. Eine chemische Raumbedingung kann beispielsweise die in dem Raum vorhandene Konzentration des Dekontaminationsmittels sein.

Ferner weist die Zentraleinheit eine Fluidleitung auf, welche ausgangsseitig mit dem Anschluss gekoppelt ist und welche derart eingerichtet ist, dass ein Fluid, welches das chemische Dekontaminationsmittel aufweist, zu dem Anschluss transferierbar ist. Dies bedeutet, dass zumindest ein Teil des chemischen Dekontaminationsmittels, welches von der Abgabeeinheit in den zu dekontaminierenden Raum abgegeben wird, von der Zentraleinheit bereit gestellt wird. Der Anschluss kann insbesondere ein Medienanschluss sein, welcher ein Teil eines geeigneten Medien-Verbindungssystems ist.

Das Medium kann ein beliebiges Fluid sein. In diesem Zusammenhang sind Flüssigkeiten und Gase ein Fluid, da sie einer beliebig kleinen Scherspannung keinen Widerstand entgegensetzen. Das Fluid kann über eine geeignete Verbindungsleitung, welche sich zwischen dem Anschluss bzw. Medienanschluss der Zentraleinheit und der Abgabeeinheit erstreckt, zu der Abgabeeinheit transferiert werden. In diesem Zusammenhang stellt der beschriebene Anschluss der Zentraleinheit einen Fluidausgang dar.

Die Fluidleitung ist ferner derart eingerichtet, dass eine externe Generatoreinheit zum Erzeugen des chemischen Dekontaminationsmittels anschließbar ist.

Die externe Generatoreinheit kann einerseits an einer Unterbrechung der Fluidleitung über die beiden durch die Unterbrechung erzeugten Enden der Fluidleitung angeschlossen werden. Andererseits kann die externe Generatoreinheit parallel zu einem Teilabschnitt der Fluidleitung geschaltet sein, wobei der Teilabschnitt optional ein oder mehrere Fluidkonditionierungskomponenten der Zentraleinheit aufweisen kann. Mittels geeigneter Ventile wie beispielsweise Dreiwegeventile oder Klappen kann die Verteilung des Volumenstroms zwischen der externen Generatoreinheit und dem Teilabschnitt der Fluidleitung eingestellt werden.

Die beschriebene Zentraleinheit stellt eine intelligente Schnittstellenanlage zwischen der externen Generatoreinheit und dem zu dekontaminierenden Raum bzw. der in oder an dem zu dekontaminierenden Raum angeordneten Abgabeeinheit dar. Dabei kann die Abgabeeinheit beispielsweise ein Begasungssystem aufweisen, so dass das chemische Dekontaminationsmittel in im Wesentlichen gasförmiger Form in den zu dekontaminierenden Raum eintritt.

Mittels der externen Generatoreinheit kann der Zentraleinheit das chemische Dekontaminationsmittel wie beispielsweise H2O2, Chlordioxid (ClO2), Ozon (03) oder andere Chemikalien zugeführt werden. Das chemische Dekontaminationsmittel kann in flüssiger und/oder in gasförmiger Form der Zentraleinheit bereitgestellt werden.

Die Möglichkeit der Verwendung einer externen Generatoreinheit hat den Vorteil, dass die Zentraleinheit mit unterschiedlichen Arten oder Typen von Generatoreinheiten betrieben werden kann. Dies gilt insbesondere für Generatoreinheiten von unterschiedlichen Herstellern. Ferner kann die Zentraleinheit und damit das gesamte Dekontaminationssystem mit verschiedenen Dekontaminationstechnologien und/oder mit verschiedenen Chemikalien betrieben werden.

Die externe Generatoreinheit kann mittels eines Medienanschlusses oder mittels mehrerer Medienanschlüsse mit der beschriebenen Zentraleinheit gekoppelt werden. Dazu kann eine geeignete Anschlusskopplung oder es können mehrere geeignete Anschlusskopplungen verwendet werden. Sofern für den Betrieb der externen Generatoreinheit eine elektrische Stromversorgung erforderlich ist, kann die entsprechende elektrische Energie optional ebenfalls von der Zentraleinheit bereitgestellt werden. Das Gleiche kann für eine Druckluftversorgung gelten, sofern eine solche für den Betrieb der externen Generatoreinheit erforderlich und/oder vorteilhaft ist. Es wird jedoch darauf hingewiesen, dass selbstverständlich auch externe Generatoreinheiten verwendet werden können, die derartige Betriebsmittel mittels geeigneter separater Anschlüsse beziehen, welche von der beschriebenen Zentrealeinheit unabhängig sind.

Gemäß einem Ausführungsbeispiel der Erfindung weist die Zentraleinheit ferner einen Fluideingang auf, welcher eingangsseitig mit der Fluidleitung gekoppelt ist und welcher zum Einspeisen von einem Fluid aus dem zu dekontaminierenden Raum in die Fluidleitung eingerichtet ist. Dies hat den Vorteil, dass ein zumindest teilweise geschlossener Fluidkreislauf zwischen der Zentraleinheit und dem zu kontaminierenden Raum aufgebaut werden kann. Das in die Fluidleitung eingespeiste Fluid kann insbesondere eine Raumluft sein, die aus dem zu dekontaminierenden Raum aufgenommen und in der Zentraleinheit auf geeignete Weise konditioniert wird. Die Raumluft kann dabei in flüssiger und/oder in gasförmiger Form das chemische Dekontaminationsmittel aufweisen, welches ggf. nach einer Aufbereitung in der Zentraleinheit über den oben genannten Anschluss und die Abgabeeinheit wieder für eine Dekontamination des Raumes verwendet werden kann.

Es wird darauf hingewiesen, dass die Fluidleitung keineswegs einen vollständig geschlossenen Kreislauf durch die Zentraleinheit hindurch bilden muss. Die Fluidleitung kann sich auch verzweigen, wobei beispielsweise über einen Fluidabzweig zumindest ein Teil des Fluids bedarfsweise an die Umgebung der Zentraleinheit abgegebenen werden kann. Die Verzweigung kann beispielsweise ein Dreiwegeventil aufweisen, so dass die Verteilung des Fluidstroms zwischen dem Fluidabzweig und der verbleibenden Fluidleitung in geeigneter Weise eingestellt werden kann. Ferner kann ein weiterer Fluidabzweig vorgesehen sein, über den Raumluft in die Fluidleitung eingespeist werden kann. Auch hier kann beispielsweise über ein Dreiwegeventil für eine geeignete Mengeneinspeisung der zugeführten Raumluft gesorgt werden.

Gemäß einem weiteren Ausführungsbeispiel der Erfindung weist die Zentraleinheit ferner eine Ventilatoreinheit auf, welche in der Fluidleitung angeordnet ist.

Mittels der Ventilatoreinheit kann in der Fluidleitung ein bestimmter Volumenstrom des Fluids bzw. des Abgabefluids hin zu dem Anschluss bzw. zu dem Fluidausgang erzeugt werden. Die Ventilatoreinheit treibt somit das Fluid und damit auch das chemische Dekontaminationsmittel an. Der Volumenstrom des Fluids kann flexibel an die jeweiligen Anforderungen angepasst werden. Der Volumenstrom des Fluids kann beispielsweise 100 m3/h bis 1000 m3/h betragen.

Die Anordnung der Ventilatoreinheit in der Fluidleitung kann dadurch realisiert sein, dass die Ventilatoreinheit in eine Unterbrechung der Fluidleitung eingeschoben ist. Der Volumenstrom durch die Fluidleitung erfolgt somit von einem ersten Teil der Fluidleitung über die Ventilatoreinheit in einen zweiten Teil der Fluidleitung.

Die Ventilatoreinheit kann einen oder mehrere Ventilatoren und/oder ggf. auch Pumpen aufweisen, welche für einen Anschub des Fluids durch die Fluidleitung sorgen. Abhängig insbesondere von dem Aggregatszustand und/oder von der Konsistenz (z. b. der Viskosität) des Fluids kann die Ventilatoreinheit in geeigneter Weise ausgestaltet sein.

Gemäß einem weiteren Ausführungsbeispiel der Erfindung weist die Zentraleinheit ferner eine interne Generatoreinheit zum Erzeugen des chemischen Dekontaminationsmittels auf, wobei die interne Generatoreinheit in der Fluidleitung angeordnet ist. Dabei kann die Generatoreinheit in Bezug zu zumindest einem Teilabschnitt der Fluidleitung in Serie oder in Parallel angeordnet sein.

Mittels der internen Generatoreinheit kann das chemische Dekontaminationsmittel wie beispielsweise H2O2, Chlordioxid (Cl02), Ozon (03) oder andere Chemikalien direkt in der Zentraleinheit erzeugt werden. Auf diese Weise wird eine weitgehend autarke Zentraleinheit geschaffen, welche auch von einer ungeübten Bedienperson sicher bedient werden kann.

Es wird darauf hingewiesen, dass auch die interne Generatoreinheit das chemische Dekontaminationsmittel in flüssiger und/oder in gasförmiger Form an die Fluidleitung bereitstellen kann.

Gemäß einem weiteren Ausführungsbeispiel der Erfindung weist die Zentraleinheit ferner eine Abbaueinheit zum zumindest teilweisen Abbauen des chemischen Dekontaminationsmittels auf, wobei die Abbaueinheit in der Fluidleitung angeordnet ist.

Durch die Abbaueinheit kann das chemische Dekontaminationsmittel, welches beispielsweise aus dem zu dekontaminierenden Raum über den oben beschriebenen Fluideingang zusammen mit der Raumluft angesaugt wird, zumindest teilweise abgebaut werden. Dadurch kann eine nachfolgende Zufuhr von chemischem Dekontaminationsmittel in das in der Fluidleitung transportierte Fluid unter definierten Bedingungen stattfinden. Auf diese Weise kann die Konditionierung des Fluids bzw. des Abgabefluids, welches an die Abgabeeinheit transferiert wird, insbesondere in Bezug auf die Konzentration an chemischem Dekontaminationsmittel, genau eingestellt und eine gleichbleibende Konditionierung gewährleistet werden.

In der Abbaueinheit kann das flüssige und/oder gasförmige Dekontaminationsmittel beispielsweise durch bekannte durch einen oder mehrere Katalysatoren beeinflusste chemische Abbaureaktionen abgebaut werden. Ferner können eine Ionisation und/oder eine elektromagnetische Bestrahlung des Fluids wie beispielsweise eine UV-Bestrahlung und/oder eine Bestrahlung mit Röntgenstrahlung zu einem erwünschten Abbau des chemischen Dekontaminationsmittels führen.

Bei einem signifikanten Abbau von dem chemischen Dekontaminationsmittel kann die Zentraleinheit somit mehrere und verschiedene Spül- und Reinigungsprozesse durchführen, wobei zumindest außerhalb des zu kontaminierenden Raumes ein geschlossener Fluidkreislauf aufrecht erhalten werden kann.

Gemäß einem weiteren Ausführungsbeispiel der Erfindung weist die Zentraleinheit ferner eine Filtereinheit auf, welche in der Fluidleitung angeordnet ist.

Die Verwendung einer in der Zentraleinheit integrierten Filtereinheit hat den Vorteil, dass insbesondere physikalische Verunreinigungen wie Staubpartikel aus dem Fluid entfernt werden können. Dadurch können ggf. stromabwärts angeordnete Einheiten zur Fluidkonditionierung vor unerwünschten Beschädigungen geschützt werden. Ferner kann ein hoher Reinheitsgrad des in den zu dekontaminierenden Raum abzugebenden Abgabefluids gewährleistet werden.

Gemäß einem weiteren Ausführungsbeispiel der Erfindung weist die Zentraleinheit ferner eine Feuchtigkeitsbeeinflussungseinheit auf, welche in der Fluidleitung angeordnet ist.

Die Feuchtigkeitsbeeinflussungseinheit kann beispielsweise eine Entfeuchtungseinheit aufweisen, so dass garantiert trockene Dekontaminationsprozesse ohne eine unerwünschte Kondensation von zumindest einem Bestandteil des Abgabefluidbildung insbesondere an den Wänden des zu dekontaminierenden Raumes entwickelt und durchgeführt werden können.

Die Feuchtigkeitsbeeinflussungseinheit kann ferner eine Befeuchtungseinheit aufweisen, welche auf vorteilhafte Weise dafür sorgen, dass das Abgabefluid nicht zu trocken ist. Zu trockene Dekontaminationsprozesse können nämlich für bestimmte Anwendungen nachteilig sein. Insbesondere kann nämlich für einige Spezialanwendungen beispielsweise der Dekontamination einer Schleuse oder einer Präparationskammer ein sog. Nassprozess vorteilhaft sein, bei dem sich an den Wänden des zu dekontaminierenden Raumes zumindest ein Bestandteil des Abgabefluids niederschlägt.

Es wird darauf hingewiesen, dass die Feuchtigkeitsbeeinflussungseinheit lediglich zumindest eine Entfeuchtungseinheit, lediglich zumindest eine Befeuchtungseinheit oder sowohl jeweils zumindest eine Entfeuchtungseinheit und eine Befeuchtungseinheit aufweisen kann. Die unterschiedlichen Einheiten können dabei innerhalb der Zentraleinheit räumlich voneinander getrennt oder in einer Bauform realisiert werden.

Gemäß einem weiteren Ausführungsbeispiel der Erfindung weist die Zentraleinheit ferner eine Temperiereinheit auf, welche in der Fluidleitung angeordnet ist.

Mittel einer beschriebenen Temperiereinheit können raumverträgliche Fluidstromtemperaturen realisiert werden. Zu diesem Zweck kann die Temperiereinheit zumindest eine Erhitzereinheit und/oder eine Kühlungseinheit aufweisen. Auch hier können unterschiedliche Einheiten räumlich voneinander getrennt oder in einer gemeinsamen Bauform angeordnet sein.

Gemäß einem weiteren Ausführungsbeispiel der Erfindung weist die Zentraleinheit ferner eine Messeinrichtung zum Messen des Volumenstroms des in der Fluidleitung geförderten Fluids auf.

Die Messeinrichtung kann mit der Steuerungseinheit gekoppelt sein, so dass im Falle eines von einem Sollwert abweichenden Volumenstroms beispielsweise die oben beschriebene Ventilatoreinrichtung entsprechend angesteuert werden kann, dass sich nachfolgend der Volumenstrom wieder auf den Sollwert einstellt.

Es wird darauf hingewiesen, dass weitere Sensoreinrichtungen verwendet werden können, um die Konditionierung des Abgabefluids in geeigneter Weise zu überwachen. Die Sensoreinrichtungen können dazu an geeigneten Stellen in der Zentraleinheit, in der Abgabeeinheit und/oder in einer Verbindungs-Fluidleitung zwischen der Zentraleinheit und der Abgabeeinheit angeordnet sein.

Der Zentraleinheit können ferner weitere Sensoreinrichtungen wie beispielsweise Temperatur- und/oder Feuchtigkeitssensoren für die Messung der Raumkonditionen während des Dekontaminationsprozesses sein. Bevorzugt werden Sensoreinrichtungen mit einem gültigen Kalibrierungszertifikat verwendet.

Im Falle der Verwendung von H2O2 als chemisches Dekontaminationsmittel können ein oder mehrere H2O2 Sensoren für die Messung der H2O2 Konzentration in dem zu dekontaminierenden Raum und/oder in dem Abgabefluid insbesondere während des Dekontaminationsprozesses bzw. des Dekontaminationszyklus verwendet werden. Auch diese Sensoren weisen bevorzugt ein aktuelles Kalibrierungszertifikat auf. Der Messbereich der H2O2 Sensoren kann beispielsweise 0ppm bis 1500ppm betragen.

Es wird darauf hingewiesen, dass auch an der Zentraleinheit ein Sensor angebracht sein kann, mit dem die Konzentration des chemischen Dekontaminationsmittels in dem Raum erfasst werden kann, in dem sich die Zentraleinheit befindet. Dadurch können Bedienpersonen bei einem unerwünschten Austritt des Dekontaminationsmittels geschützt werden. Im Falle der Verwendung von H2O2 als chemisches Dekontaminationsmittel kann beispielsweise ein (kalibrierter) H2O2 Sensor mit einem Messbereich von 0ppm bis 3ppm verwendet werden. Ferner kann zur Messung der maximalen Arbeitsplatz-Konzentration am Ende oder nach einem Dekontaminationsprozess ein H2O2 Sensor beispielsweise der Fa. Dräger verwendet werden, welcher mit einem integrierten Pumpenmodul ausgestattet ist.

Sämtliche Sensoren können Signale mit einem Signalstrompegel von 4mA bis 20mA ausgeben. Um eine hohe Betriebssicherheit zu gewährleisten können einige oder alle der verwendeten Sensoren drahtbruchüberwacht und/oder in bekannter Weise mittels Messtrennklemmen verdrahtet sein.

Gemäß einem weiteren Ausführungsbeispiel der Erfindung weist die Zentraleinheit ferner einen weiteren Anschluss zum Anschließen einer weiteren Abgabeeinheit auf, welche zum Abgeben eines chemischen Dekontaminationsmittels in einen weiteren zu dekontaminierenden Raum eingerichtet ist. Ferner ist die Steuerungseinheit eingerichtet zum Steuern einer Zufuhr des chemischen Dekontaminationsmittels zu der weiteren Abgabeeinheit.

Mit der beschriebenen Zentraleinheit können somit zwei unterschiedliche Räume dekontaminiert werden. Die in den unterschiedlichen Räumen durchzuführenden Dekontaminationsprozesse können dabei gleich oder unterschiedlich sein. Dabei kann für beide Räume das gleiche oder unterschiedliche Dekontaminationsmittel verwendet werden. Ebenfalls können gleiche oder unterschiedlich konzentrierte Dekontaminationsmittel verwendet werden.

Im Falle von gleichen Dekontaminationsprozessen können beide Prozesse gleichzeitig durchgeführt werden. Dabei kann für das Abgabefluid, welches mittels der Abgabeeinheit und der weiteren Abgabeeinheit abgegeben wird, eine für beide Räume geeignete Abgabefluidkonditionierung gewählt werden. Im Falle von unterschiedlichen Dekontaminationsprozessen müssen diese Prozesse zeitlich voneinander getrennt ablaufen, um für jede Abgabeeinheit eine optimal angepasste Abgabefluidkonditionierung zu gewährleisten, welche unabhängig von der Konditionierung des Abgabefluids für die andere Abgabeeinheit ist. Dies gilt jedenfalls solange, wie die entsprechenden Komponenten der Zentraleinheit für eine Abgabefluidkonditionierung lediglich in einfacher Ausfertigung vorhanden sind.

Mit der beschriebenen zumindest zwei Anschlüsse aufweisenden Zentraleinheit können somit mehrere Räume bzw. Kammern, Geräte oder Anlagen dekontaminiert werden. Im Falle von einem einheitlichen Dekontaminationsprozess können die Abgabeeinheiten, die verschiedenen Räume zugeordnet sind, in Serie oder Parallel geschalten werden.

Die Steuerungseinheit kann somit mehrere Dekontaminationsanwendungen gemeinsam oder alternativ unabhängig voneinander steuern. Sämtliche Dekontaminationsanwendungen können durch eine geeignete Abgabefluid- bzw. Fluidkonditionierung flexibel an die jeweiligen Bedürfnisse angepasst und von einem Benutzer durch eine geeignete Informationseingabe an der Benutzerschnittstelle individuell eingestellt werden.

Es wird darauf hingewiesen, dass die Zentraleinheit auch noch weitere Anschlüsse aufweisen kann, so dass auch mehr als zwei Abgabeeinheiten an die Zentraleinheit angeschlossen und eine entsprechende Anzahl an Räumen mit der beschriebenen Zentraleinheit dekontaminiert werden kann.

Gemäß einem weiteren Ausführungsbeispiel der Erfindung weist die Zentraleinheit ferner eine erste Verteileinheit auf, welche einen Eingang, einen ersten Ausgang und einen zweiten Ausgang aufweist. Dabei ist der Eingang mit einem Ausgang der Fluidleitung gekoppelt, der erste Ausgang ist mit dem Anschluss zum Anschließen der Abgabeeinheit gekoppelt und der zweite Ausgang ist mit dem weiteren Anschluss zum Anschließen der weiteren Abgabeeinheit gekoppelt.

Die Verteileinheit kann eine Verzweigung aufweisen, die beispielsweise mittels eines Dreiwegeventils realisiert sein kann. Die Verteilereinheit kann mit der Steuerungseinheit gekoppelt sein, so dass die Verteilung der Volumenströme des Fluids bzw. des Abgabefluids automatisch und in geeigneter Weise eingestellt werden kann. Insbesondere kann bei unterschiedlichen Dekontaminationsprozessen in einer ersten Zeitspanne die Verteileinheit lediglich einen Fluidstrom zu dem Anschluss (zum Anschließen der Abgabeeinheit) zulassen, wohingegen in einer zweiten Zeitspanne, welche zeitlich von der ersten Zeitspanne separiert ist, von der Verteileinheit lediglich ein Fluidstrom zu dem weiteren Anschluss (zum Anschließen der weiteren Abgabeeinheit) zugelassen wird.

Es wird darauf hingewiesen, dass im Falle der Verwendung von mehreren Abgabeeinheiten die erste Verteileinheit eine entsprechende Anzahl von Ausgängen ausweisen kann, so dass alle Abgabeeinheiten individuell mit Fluid versorgt werden können.

Gemäß einem weiteren Ausführungsbeispiel der Erfindung weist die Zentraleinheit ferner eine zweite Verteileinheit auf, welche einen ersten Eingang, einen zweiten Eingang und einen Ausgang aufweist. Dabei ist der erste Eingang dem zu dekontaminierenden Raum zugeordnet, der zweite Eingang ist dem weiteren zu dekontaminierenden Raum zugeordnet und der Ausgang ist mit einem Eingang der Fluidleitung gekoppelt. Über den ersten Eingang kann somit Raumluft aus dem zu dekontaminierenden Raum und über den zweiten Eingang kann Raumluft aus dem weiteren zu dekontaminierenden Raum angesaugt werden.

Auch die zweite Verteileinheit kann eine Verzweigung aufweisen, die beispielsweise mittels eines Dreiwegeventils realisiert sein kann. Ferner kann auch die zweite Verteileinheit mit der Steuerungseinheit gekoppelt sein, so dass in entsprechender Weise wie bei der ersten Verteileinheit die Volumenströme von den beiden zu dekontaminierenden Räumen eingestellt werden können. Bei einem Dekontaminationsprozess für den zu dekontaminierenden Raum, bei dem der erste Ausgang der ersten Verteileinheit geöffnet und der zweite Ausgang der ersten Verteileinheit geschlossen ist, ist bevorzugt der erste Eingang der zweiten Verteileinheit geöffnet und der zweite Eingang der zweiten Verteileinheit geschlossen. Entsprechendes gilt mit umgekehrten "Vorzeichen" für einen Dekontaminationsprozess für den weiteren zu dekontaminierenden Raum.

Es wird darauf hingewiesen, dass im Falle der Dekontamination von mehreren Räumen die zweite Verteileinheit ein entsprechende Anzahl von Eingängen ausweisen kann, so dass aus allen zu dekontaminierenden Räumen Raumluft abgesaugt werden kann.

Gemäß einem weiteren Aspekt der Erfindung wird ein Dekontaminationssystem zum chemischen Dekontaminieren von Räumen beschrieben. Das beschriebene Dekontaminationssystem weist auf (a) eine Zentraleinheit des oben beschriebenen Typs und (b) eine Abgabeeinheit zum Abgeben eines chemischen Dekontaminationsmittels in den zu dekontaminierenden Raum. Dabei sind die Zentraleinheit und die Abgabeeinheit über eine Verbindungsleitung miteinander gekoppelt.

Auch dem beschriebenen Dekontaminationssystem liegt die Erkenntnis zugrunde, dass ein Dekontaminationsprozess, welcher von der Abgabeeinheit in dem zu dekontaminierenden Raum ausgeführt wird, von der Steuereinheit der Zentraleinheit entsprechend einer benutzerdefinierten Anweisung durchgeführt werden kann.

Die Abgabeeinheit kann beispielsweise eine sog. Düseneinheit sein, welche das chemische Dekontaminationsmittel in dem zu dekontaminierenden Raum fein versprüht und somit zu einer effektiven und wirksamen Verteilung des Dekontaminationsmittels beiträgt. Die Verteilung des Dekontaminationsmittels kann beispielsweise durch Ventilatoren, welche die Raumluft in dem betreffenden Raum verwirbeln, unterstützt werden.

Wie bereits oben erläutert kann die Steuerungseinheit derart eingerichtet sein, dass validierte Prozesse durch eine geeignete Aktivierung von Komponenten der Zentraleinheit abgefahren werden können. Dabei kann das Abgabefluid, welches zumindest das chemische Dekontaminationsmittel in flüssiger und/oder gasförmiger Form aufweist, durch zumindest eine aktivierte Komponente in geeigneter Weise konditioniert werden. Abhängig von den jeweiligen Bedürfnissen kann das Abgabefluid beispielsweise erwärmt, abgekühlt, befeuchtet, getrocknet, katalysiert, ionisiert, mit ultraviolettem (UV) Licht bestrahlt und/oder mit einem Über- oder einem Unterdruck beaufschlagt werden. Ferner kann der Anteil an dem Dekontaminationsmittel in dem Abgabefluid durch ein Konzentrieren oder ein Verdünnen erhöht bzw. erniedrigt werden. Somit können mit der Zentraleinheit in Hinblick auf verschiedenste Anwendungsfälle geeignete Abgabefluidkonditionen geschaffen werden.

Die Verbindungsleitung kann lediglich eine Steuerleitung sein. In diesem Fall wird der Abgebeinheit das chemische Dekontaminationsmittel von einer anderen Einheit des Dekontaminationssystems zugeführt. Die Verbindungsleitung kann jedoch auch eine Medienleitung sein, welche einen Transfer von einem Fluid bzw. einem Abgabefluid zwischen der Zentraleinheit und der Abgabeeinheit ermöglicht.

Gemäß einem Ausführungsbeispiel der Erfindung ist die Abgabeeinheit eine mobile Abgabeeinheit oder eine stationäre Abgabeeinheit.

Eine mobile Abgabeeinheit hat den Vorteil, dass die Dekontamination in verschiedenen Räumen durchgeführt werden kann. Auch lange Gänge mit einer Länge von beispielsweise bis zu 40m können durch ein geeignetes Bewegen der mobilen Abgabeeinheit auf effektive Weise dekontaminiert werden.

Eine stationäre Abgabeeinheit kann beispielsweise in einem Installationsleitungssystem integriert sein. Das Installationsleitungssystem kann beispielsweise ein infrastrukturelles Raumlüftungssystem sein, in welches die stationäre Abgabeeinheit eingebunden ist.

Gemäß einem weiteren Aspekt der Erfindung wird ein Verfahren zum chemischen Dekontaminieren von Räumen beschrieben. Das beschriebene Verfahren weist auf (a) ein Eingeben von zumindest einer Information an eine Steuerungseinheit einer Zentraleinheit mittels einer mit der Steuerungseinheit gekoppelten Benutzerschnittstelle der Zentraleinheit, wobei die Information mit einem benutzerdefinierten Dekontaminationsprozess verknüpft ist, (b) ein Erzeugen des chemischen Dekontaminationsmittels mittels einer in Bezug auf die Zentraleinheit externen Generatoreinheit, (c) ein Transferieren des chemischen Dekontaminationsmittels zu einem Anschluss der Zentraleinheit mittels einer Fluidleitung, an welcher die externe Generatoreinheit angeschlossen ist, (d) ein Abgeben eines chemischen Dekontaminationsmittels in einen zu dekontaminierenden Raum mittels einer Abgabeeinheit, welche über den Anschluss mit der Zentraleinheit gekoppelt ist, und (e) ein Steuern einer Zufuhr des chemischen Dekontaminationsmittels zu der Abgabeeinheit mittels der Steuerungseinheit, wobei die Steuerungseinheit derart eingerichtet ist, dass basierend auf der Information der entsprechende benutzerdefinierte Dekontaminationsprozess von der Zentraleinheit durchgeführt wird. Die externe Generatoreinheit ist an durch eine Unterbrechung der Fluidleitung erzeugte Enden angeschlossen oder die externe Generatoreinheit ist parallel zu einem Teilabschnitt der Fluidleitung geschaltet.

Dem beschriebenen Verfahren liegt die Erkenntnis zugrunde, dass ein Dekontaminationsprozess, welcher von der Abgabeeinheit in dem zu dekontaminierenden Raum ausgeführt wird, von der Steuereinheit der Zentraleinheit entsprechend einer benutzerdefinierten Anweisung durchgeführt werden kann. Die Zentraleinheit und die Abgabeeinheit stellen dabei wichtige Komponenten eines Dekontaminationssystems dar, bei dem die Abgabeeinheit räumlich getrennt von der Zentraleinheit angeordnet sein kann. Durch die Möglichkeit der Verwendung einer externen Generatoreinheit kann die Zentraleinheit auf einfache und effiziente Weise mit unterschiedlichen Arten oder Typen von Generatoreinheiten betrieben werden. Dies gilt insbesondere für Generatoreinheiten von unterschiedlichen Herstellern. Ferner kann die Zentraleinheit und damit das gesamte Dekontaminationssystem mit verschiedenen Dekontaminationstechnologien und/oder mit verschiedenen Chemikalien betrieben werden.

Wie bereits oben erläutert kann mittels der externen Generatoreinheit der Zentraleinheit das chemische Dekontaminationsmittel wie beispielsweise H2O2, Chlordioxid (ClO2), Ozon (03) oder andere Chemikalien zugeführt werden. Das chemische Dekontaminationsmittel kann in flüssiger und/oder in gasförmiger Form der Zentraleinheit bereitgestellt werden.

Das beschriebene Dekontaminationsverfahren kann einen sog. Nassprozess umfassen, bei dem eine gewollte Kondensation in dem zu dekontaminierenden Raum auftritt. Die Kondensation kann dabei bevorzugt an den Wänden des zu kontaminierenden Raumes auftreten. Im Falle eines Nassprozesses kann der zu dekontaminierende Raum insbesondere eine Schleuse oder eine Präparationskammer bzw. ein sog. Isolator sein.

Das Dekontaminationsverfahren kann ferner eine von der Abgabeeinheit durchgeführte Desinfektion aufweisen, welche beispielsweise durch Sprühen realisiert werden kann. Dabei kann eine gleichmäßige Verteilung von dem chemischen Dekontaminationsmittel in dem zu dekontaminierenden Raum beispielsweise durch geeignete Ventilatoren und/oder Düsen unterstützt werden.

Es wird darauf hingewiesen, dass Ausführungsformen der Erfindung mit Bezug auf unterschiedliche Erfindungsgegenstände beschrieben wurden. Insbesondere sind einige Ausführungsformen der Erfindung mit Vorrichtungsansprüchen und andere Ausführungsformen der Erfindung mit Verfahrensansprüchen beschrieben. Dem Fachmann wird jedoch bei der Lektüre dieser Anmeldung sofort klar werden, dass, sofern nicht explizit anders angegeben, zusätzlich zu einer Kombination von Merkmalen, die zu einem Typ von Erfindungsgegenstand gehören, auch eine beliebige Kombination von Merkmalen möglich ist, die zu unterschiedlichen Typen von Erfindungsgegenständen gehören.

Weitere Vorteile und Merkmale der vorliegenden Erfindung ergeben sich aus der folgenden beispielhaften Beschreibung derzeit bevorzugter Ausführungsformen. Die einzelnen Figuren der Zeichnung dieser Anmeldung sind lediglich als schematisch und als nicht maßstabsgetreu anzusehen.

Die Figur 1 zeigt in einer schematischen Darstellung eine Zentraleinheit, welche mit einer in einen zu dekontaminierenden Raum befindlichen Abgabeeinheit gekoppelt ist.

Die Figuren 2a und 2b zeigen verschiedenen Ansichten der in Figur 1 schematisch dargestellten Zentraleinheit.

An dieser Stelle wird darauf hingewiesen, dass sich in der Zeichnung die Bezugszeichen von gleichen oder von einander entsprechenden Komponenten lediglich in ihrer ersten Ziffer voneinander unterscheiden.

Ferner wird darauf hingewiesen, dass die nachfolgend beschriebene Ausführungsform lediglich eine beschränkte Auswahl an möglichen Ausführungsvarianten der Erfindung darstellt.

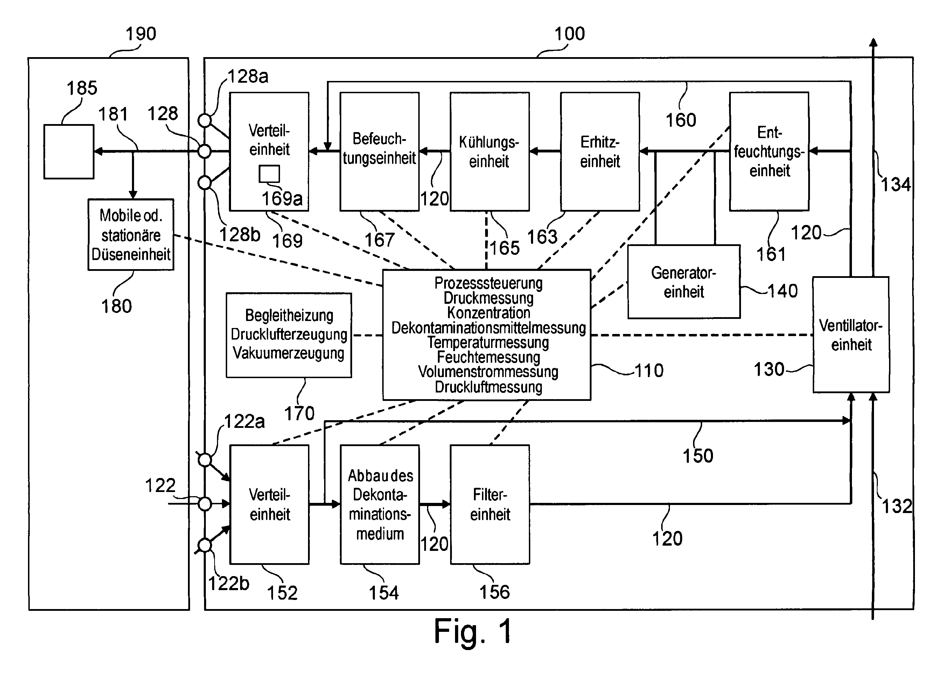

Die Figur 1 zeigt in einer schematischen Darstellung eine Zentraleinheit 100 gemäß einem bevorzugten Ausführungsbeispiel der Erfindung. Die Zentraleinheit 100 ist über einen Anschluss 128, welcher einen Fluidausgang darstellt, und über eine Verbindungsleitung 181 mit einer Abgabeeinheit 180 gekoppelt. Die Abgabeeinheit 180 befindet sich in einem zu dekontaminierenden Raum 190. Gemäß dem hier dargestellten Ausführungsbeispiel ist die Abgabeeinheit 180 eine mobile oder stationäre Düseneinheit 180, welche ein Fluid in dem Raum 190 verteilt. Das Fluid, welches auch als Abgabefluid bezeichnet wird, weist ein chemisches Dekontaminationsmittel auf. Das Fluid wird von der Zentraleinheit 100 an die Abgabeeinheit 180 über die Verbindungsleitung 181 transferiert. Gemäß dem hier dargestellten Ausführungsbeispiel befindet sich in dem zu dekontaminierenden Raum 190 ferner ein Lüftungssystem 185, welchem ebenfalls von der Zentraleinheit das Fluid zur Verfügung gestellt wird und welches ebenfalls dazu beiträgt, dass das Fluid möglichst gleichmäßig in dem zu dekontaminierenden Raum 190 verteilt wird. Es wird jedoch ausdrücklich darauf hingewiesen, dass die Verwendung des Lüftungssystems 185 optional ist.

Die Zentraleinheit 100 weist eine zentrale Steuerungseinheit 110 auf, welche über eine in Figur 1 nicht dargestellte Benutzerschnittstelle gekoppelt ist. Über die Benutzerschnittstelle kann eine Bedienperson zumindest eine Information an die Steuerungseinheit 110 übergeben, wobei die zumindest eine Information mit einem benutzerdefinierten Dekontaminationsprozess verknüpft ist, welcher, wie nachfolgend noch erläutert wird, von der Zentraleinheit 100 in Verbindung mit der Abgabeeinheit 180 durchgeführt wird. Dies bedeutet, dass die Steuerungseinheit 110 derart eingerichtet ist, dass basierend auf der Information der entsprechende benutzerdefinierte Dekontaminationsprozess durchführbar ist.

Gemäß dem hier dargestellten Ausführungsbeispiel weist die Zentraleinheit 100 ferner eine Fluidleitung 120 auf, welche sich von einem Fluideingang 122 und zwei weiteren Fluideingängen 122a und 122b hin zu dem Fluidausgang 128 und zwei weiteren Anschlüssen bzw. Fluidausgängen 128a und 128b erstreckt. Die beiden weiteren Fluidausgänge 128a und 128b können mit jeweils einer nicht dargestellten weiteren Abgabeeinheit gekoppelt werden. Die weiteren Abgabeeinheiten können sich in dem zu kontaminierenden Raum 190 oder in nicht dargestellten weiteren zu kontaminierenden Räumen befinden. In entsprechender Weise können die weiteren Fluideingänge 122a und 122b den nicht dargestellten weiteren zu kontaminierenden Räumen zugeordnet sein, so dass auch aus diesen Räumen die jeweilige Raumluft, welche üblicherweise auch das chemische Dekontaminationsmittel aufweist, abgesaugt werden kann.

Die dargestellte Zentraleinheit 100 weist ferner eine ausgangsseitig angeordnete Verteileinheit 169 auf, welche mit der Steuerungseinheit 110 gekoppelt ist und welche einen Volumenstrom des in der Fluidleitung geförderten Fluids beliebig auf die verschiedenen Fluidausgänge 128, 128a und 128b verteilen kann. Außerdem weist die dargestellte Zentraleinheit 100 eine eingangsseitig angeordnete Verteileinheit 152 auf, welche die verschiedenen Fluideingänge 122, 122a und 122b mit der Fluidleitung koppelt. Auch die Verteileinheit 152 ist mit der Steuerungseinheit 110 gekoppelt, so dass die Verteilung der Volumenströme, die über die verschiedenen Fluideingänge 122, 122a und 122b in die Fluidleitung 120 eingespeist werden, beliebig eingestellt werden kann.

Es wird darauf hingewiesen, dass die Erfindung auch mit lediglich dem einen Fluidausgang 128 und dem einen Fluideingang 122 realisiert werden kann, so dass sowohl die beiden Verteileinheiten 169 und 152 als auch die weiteren Fluidausgänge 128a und 128b und die weiteren Fluideingänge 122a und 122b als optional anzusehen sind.

In der Fluidleitung 120 befindet sich eine Ventilatoreinheit 130, welche einen Ventilator oder mehrere Ventilatoren aufweist und welche das in der Fluidleitung 120 befindliche Fluid in Richtung der Fluidausgänge 128, 128a und 128b treibt. Der Volumenstrom durch die Fluidleitung kann mittels der Steuerungseinheit 110 eingestellt werden, welche auch mit der Ventilatoreinheit 130 über eine gestrichelt dargestellte Steuerleitung gekoppelt ist. Gemäß dem hier dargestellten Ausführungsbeispiel ist das Fluid gasförmig. Es weist Luft und das chemische Dekontaminationsmittel auf. Es wird jedoch darauf hingewiesen, dass das Fluid auch zumindest teilweise flüssig sein kann. In diesem Fall müssen die Ventilatoreinheit entsprechend angepasst werden, wobei der oder die Ventilatoren beispielsweise durch eine oder mehrere Pumpen ersetzt werden können.

Wie aus Figur 1 ersichtlich, ist ferner eine Zuluftleitung 132 vorhanden, über welche Raumluft aus dem Raum, in dem sich die Zentraleinheit 100 befindet, in die Ventilatoreinheit eingespeist werden kann. Ferner ist eine Abluftleitung 134 vorhanden, über welche das in dem unteren Teil der Fluidleitung 120 transportierte Fluid zumindest teilweise an die Umgebung des Ventilatoreinheit 130 abgegeben werden kann. Die entsprechenden Volumenströme und insbesondere (a) das Verhältnis zwischen den in die Ventilatoreinheit 130 eingehenden Volumenströmen (a1) aus dem unteren Teil der Fluidleitung 120 und (a2) aus der Zuluftleitung 132 und (b) das Verhältnis zwischen den ausgehenden Volumenströmen (b1) in den oberen Teil der Fluidleitung 120 und (b2) in die Abluftleitung 134 können ebenfalls von der Steuerungseinheit 110 vorgegeben werden. So ist es beispielsweise denkbar, dass die gesamte Fluidmenge, welche über den unteren Teil der Fluidleitung 120 der Ventilatoreinheit 130 zugeführt wird, in die Abluftleitung 134 eingespeist wird und dass die gesamte Fluidmenge, welche in den oberen Teil der Fluidleitung 120 transferiert wird, aus der Zuluftleitung 132 entnommen wird.

Der Verteileinheit 152 sind eine Abbaueinheit 154 und eine Filtereinheit 156 nachgeschaltet. Mittels der Abbaueinheit 154 kann das flüssige und/oder gasförmige Dekontaminationsmittel beispielsweise durch einen oder mehrere Katalysatoren chemische abgebaut werden. Ferner können eine Ionisation und/oder eine UV-Bestrahlung des Fluids zu einem Abbau des chemischen Dekontaminationsmittels führen. Mittels der Filtereinheit 156 kann das durch den unteren Teil der Fluidleitung 120 transportierte Fluid gefiltert und somit von physikalischen, chemischen und/oder biologischen Verunreinigungen wie beispielsweise Staubpartikel, Sporen, Viren etc. gereinigt werden.

Gemäß dem hier dargestellten Ausführungsbeispiel ist ferner eine erste Bypassleitung 150 vorgesehen, über welche das von den Fluideingängen 122, 122a und 122b eingespeiste Fluid unter Umgehung der für eine Fluidreinigung vorgesehen Komponenten Abbaueinheit 154 und Filtereinheit 156 direkt an die Ventilatoreinheit 130 übergeben werden kann.

Wie aus Figur 1 ersichtlich, ist der Ventilatoreinheit 130 eine Entfeuchtungseinheit 161 nachgeschaltet, mittels welcher das Fluid bei Bedarf befeuchtet werden kann. Der Betrieb der Entfeuchtungseinheit 161 kann ebenfalls von der Steuerungseinheit 110, welche auch mit der Entfeuchtungseinheit 161 über eine gestrichelt dargestellte Steuerungsleitung verbunden ist, gesteuert oder bei Verwendung von in Figur 1 nicht dargestellten Feuchtigkeitssensoren geregelt werden.

Gemäß dem hier dargestellten Ausführungsbeispiel ist der Entfeuchtungseinheit 161 eine Generatoreinheit 140 zum Erzeugen des chemischen Dekontaminationsmittels nachgeschaltet. Wie aus Figur 1 ersichtlich, ist die Generatoreinheit 140 parallel zu einem Abschnitt eines in Figur 1 dargestellten oberen Teils der Fluidleitung 120 geschaltet. Auch hier können die Volumenströme durch die Generatoreinheit 140 und durch den genannten Abschnitt der Fluidleitung 120 durch nicht dargestellte Stellelemente über die Steuerungseinrichtung 110 frei eingestellt werden.

Gemäß dem hier dargestellten Ausführungsbeispiel ist die Generatoreinheit 140 ein sog. H2O2 Generator. Dies bedeutet, dass H2O2 das chemische Dekontaminationsmittel darstellt. Die Kommunikation zwischen der Steuereinheit 110 und dem H2O2 Generator kann mittels eines Bussystems erfolgen. Dazu können an der nicht dargestellten Benutzerschnittstelle beispielsweise über einen berührungsempfindlichen Bildschirm alle prozessrelevanten Daten bzw. Messwerte sowie Status und Störmeldungen vom H2O2 Generator angezeigt werden.

Die Zentraleinheit weist ferner eine Erhitzereinheit 163 und eine Kühlungseinheit 165 auf. Diese beiden Einheiten 163 und 165 können auch in einer Temperiereinheit zusammengefasst werden. Mittels der Erhitzereinheit 163 und der Kühlungseinheit 165 können raumverträgliche Fluidstromtemperaturen realisiert werden.

Es wird darauf hingewiesen, dass je nach Anwendungsfall auch eine der beiden Einheiten 163 oder 165 weggelassen werden kann. Sollte es beispielsweise nicht erforderlich sein, eine Kühlung des Fluids vorzunehmen, dann kann die Kühlungseinheit 165 auch weggelassen werden. Mittels der Steuerungseinheit 110 kann sowohl der Betrieb der Erhitzereinheit 163 als auch der Betrieb der Kühlungseinheit 165 in geeigneter Weise gesteuert oder bei Verwendung von entsprechenden nicht dargestellten Temperatursensoren auch geregelt werden.

Der Kühlungseinheit 165 ist eine Befeuchtungseinheit 167 nachgeschaltet. Mittels der Befeuchtungseinheit 167, deren Betrieb ebenfalls von der Steuerungseinheit 110 gesteuert werden kann, kann dafür gesorgt werden, dass das Fluid, welches an die Abgabeeinheit(en) transferiert wird, nicht zu trocken ist. Auch dies kann für einige Dekontaminationsanwendungen vorteilhaft sein.

Gemäß dem hier dargestellten Ausführungsbeispiel ist ferner eine zweite Bypassleitung 160 vorgesehen, über welche unter Umgehung der für eine Fluidkonditionierung vorgesehen Komponenten Entfeuchtungseinheit 161, Generatoreinheit 140, Erhitzereinheit 163, Kühlungseinheit 165 und Befeuchtungseinheit 167 das Fluid direkt von der Ventilatoreinheit 130 an die Fluidausgänge 128, 128a und 128b transferiert werden kann. Auch der Volumenstrom durch die zweite Bypassleitung 160 kann von der Steuerungseinheit 110 durch eine geeignete Ansteuerung von nicht dargestellten Stellelementen wie beispielsweise Ventile oder Klappen gesteuert oder sogar geregelt werden.

Wie ferner aus Figur 1 ersichtlich, weist die dargestellte Zentraleinheit 100 ferner eine Volumenstrom-Messeinrichtung 169a auf. Dies erfasst den Volumenstrom des an zumindest einen der Fluidausgänge 128, 128a und 128b transferierten Fluidstroms. Gemäß dem hier dargestellten Ausführungsbeispiel ist die Messeinrichtung 169a der Verteileinheit 169 zugeordnet. Die Messeinrichtung 169a kann den gesamten Volumenstrom durch den oberen Teil der Fluidleitung 120 erfassen oder optional auch mehrere Messsensoren aufweisen, welche jeweils individuell den Volumenstrom zu den einzelnen Fluidausgängen 128, 128a und 128b erfassen. Die Messeinrichtung 169a ist über eine geeignete aus Gründen der Übersichtlichkeit nicht dargestellte Messleitung mit der Steuerungseinheit 110 verbunden, so dass die Steuerungseinheit 110 stets über den aktuellen Volumenstrom bzw. die aktuellen Volumenströme informiert ist.

Gemäß dem hier dargestellten Ausführungsbeispiel weist die Zentraleinheit 110 ferner eine sog. Hilfseinheit 170 auf, welche ebenfalls über eine gestrichelt dargestellte Steuerleitung mit der Steuerungseinheit 110 verbunden ist. Die Hilfseinheit 170 kann beispielsweise eine Begleitheizeinrichtung, eine Drucklufterzeugungseinrichtung und/oder eine Vakuum- bzw. Unterdruckerzeugungseinrichtung aufweisen. Diese Einrichtungen können mit den oben ausführlich beschriebenen Komponenten der Zentraleinheit, welche in dem unteren Teil oder in bzw. an dem oberen Teil der Fluidleitung 120 angeordnet sind, gekoppelt sein und deren Betrieb in geeigneter Weise unterstützen. So kann beispielsweise die Begleitheizeinrichtung eine oder mehrere Heizelemente wie beispielsweise Heizmatten aufweisen, welche um zumindest einen Abschnitt der Fluidleitung 120 gelegt sind und so zu einer Erwärmung des Fluids führen können.

Es wird darauf hingewiesen, dass die hier beschriebene Ausführungsform der Zentraleinheit 100 derzeit eine sog. Vollversion darstellt, welche alle derzeit als relevant betrachteten Komponenten für eine Fluidreinigung und/oder eine Fluidkonditionierung aufweist. Abhängig von der jeweiligen Dekontaminationsanwendung ist es jedoch auch möglich, auf zumindest eine der in Figur 1 dargestellten Komponenten zu verzichten und somit eine preiswerte abgespeckte Version der Zentraleinheit anzubieten. In diesem Sinne ist jede der in Figur 1 dargestellten Komponenten als für die vorliegende Erfindung optional anzusehen. Ebenso ist es jedoch auch denkbar, dass in Zukunft noch weitere Komponenten der Zentraleinheit 100 hinzugefügt werden, welche ggf. für spezielle Dekontaminationsanwendungen vorteilhaft sind.

Im Folgenden wird noch auf ein bevorzugtes Dekontaminationsverfahren eingegangen, welches mit der beschriebenen Zentraleinheit in Verbindung mit einer Abgabeeinheit durchgeführt werden kann.

### A) Prozess 1: Konditionierung

Die Luft, welche oben auch allgemein als Fluid bezeichnet wurde, wird aus dem zu dekontaminierenden Raum über einen Schlauch oder ein Rohrsystem mittels der in der Zentraleinheit 100 integrierten Ventilatoreinheit angesaugt. Über die Bypassleitung 150 wird die angesaugte Luft direkt der Ventilatoreinheit 130 zugeführt und von dort in die Entfeuchtungseinheit 161 geleitet. Die Entfeuchtungseinheit 161, welche beispielsweise einen Sorptionsentfeuchter aufweisen kann, wird der Raumluft Feuchtigkeit entzogen und nachfolgend in der Erhitzereinheit 163 erwärmt. Die Raumluft kann dann vergleichsweise trocken mit einer relativen Luftfeuchtigkeit von beispielsweise ca. 20% wieder in den zu dekontaminierenden Raum eingeblasen werden. Die Kühlungseinheit 165 sowie die Befeuchtungseinheit 167 werden in dieser Phase nicht aktiviert. Die Luftmenge und die Luftkondition (Temperatur und Feuchte) regelt die Zentraleinheit 100 nach validierten Prozessen. Die Ansaugluft wird permanent gemessen und überwacht. Dies kann sowohl für die Menge als auch für die Qualität der Ansaugluft gelten.

### B) Prozess 2: Dekontamination

In den Luftkreislauf aus Prozess 1 wird ein H2O2 Gemisch aus der Generatoreinheit 140 beigemischt. Die Generatoreinheit 140 hat lediglich die Aufgabe H2O2 Gas zu erzeugen. Bei einer relative hohen Umluftmenge (ca. 100 bis 1000m3/h) und einer geringen H2O2 Gasbeimischung (ca. 20-200 m3/h mit ca. 1000 bis 2000ppm H2O2 bei 80 - 90°C) ergibt sich nach derzeitigem Stand eine besonders gute Mischluft. Die Temperatur kann produktoptimiert und/oder raumoptimiert gesteuert werden. Die H2O2 Gaskonzentration kann gut dosiert werden und unerwünschte Kondensationen im zu dekontaminierenden Raum können infolge der oben beschriebenen Entfeuchtung verhindert werden. Durch die hohe, variable Luftmenge kann eine hochturbulente und tiefenwirksame Raumspülung erreicht werden.

Außerdem können durch die hohe, variable Luftmenge Raumdekontaminationen auch mittels stationärer Einbringsysteme wie z. b. Lüftungsanlagen durchgeführt werden.

Die Gaskonzentration in der Ausgangsluft kann einen brauchbaren Referenzwert für den Raumzustand in dem zu dekontaminierenden Raum darstellen. Über eine in der Hilfseinrichtung 170 integrierte Druckluft-Kompressoranlage kann eine mobile Begasungsanlage betrieben werden und/oder einzelne Kammern in einen Überdruck oder Vakuumzustand gebracht werden. Dadurch kann in bestimmten Fällen der Dekontaminationsprozess erheblich verbessert und/oder verkürzt werden.

Im Falle einer Dekontamination mit Nasschemie können in den Umluftkreislauf zwischen dem zu dekontaminierenden Raum 190 und der Zentraleinheit 100 Chemikalien wie beispielsweise Ammoniak, Lösemittel, Desinfektionsmittel etc. eingesprüht und zerstäubt werden.

### C) Prozess 3: Reinigung/Abbau

Nach Beendigung des Dekontaminationsprozesses kann der H2O2 Gehalt in der Raumluft und in der Oberflächenbenetzung durch einen Spülbetrieb abgebaut werden. Dieser Prozess ist in der Regel der zeitintensivste Prozess im gesamten Dekontaminationszyklus. Die erhöhte Umluftmenge wird in der Zentraleinheit 100 über die Abbaueinheit 154 geführt, welche einen Katalysator aufweisen kann. Ferner kann die Umluft noch in der Erhitzereinheit 163 erwärmt und hocheffizient in den Raum eingeblasen werden. Für den H2O2- oder allgemein den Chemie Abbau können zum Katalysator oder anstatt dem Katalysator andere Systeme wie Bestrahlungssysteme oder Ionisationssysteme eingesetzt oder dazu geschalten werden. Mit der Zentraleinheit 100 können auch verschiedene Lüftungsanlagen betrieben werden. Die Luftzustände können permanent gemessen und überwacht werden.

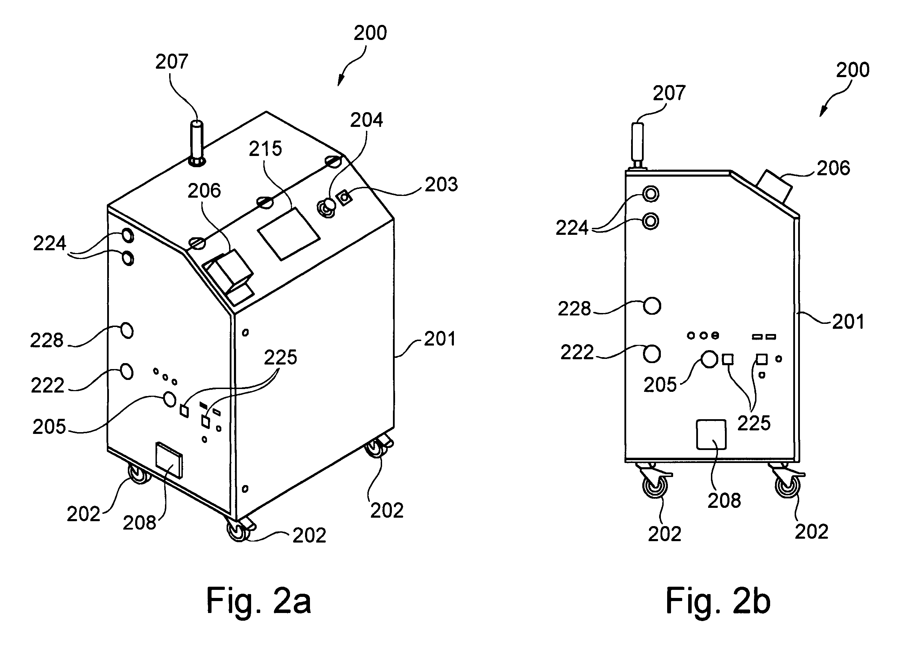

Die Figuren 2a und 2b zeigen verschiedenen Ansichten der in Figur 1 schematisch dargestellten Zentraleinheit, welche nun mit dem Bezugszeichen 200 versehen ist. Gemäß dem hier dargestellten Ausführungsbeispiel ist die Zentraleinheit 200 als mobile Anlage ausgeführt. Demzufolge sind an einem Chassis bzw. einem Gehäuse 201 der Zentraleinheit 200 Rollen 202 angebracht, so dass die Zentraleinheit 200 auf einfache Weise verfahren werden kann. Es wird jedoch darauf hingewiesen, dass die Zentraleinheit auch als stationäre Einheit realisiert werden kann. Das Chassis 201 ist ein rundum geschlossenes Gehäuse mit Wartungstüren und verschiedenen Einbauten.

Wie aus Figur 2 ersichtlich, weist die Zentraleinheit 200 einen Hauptschalter 203 und einen Notausschalter 204 auf. Über eine Steckdose 205 kann die Zentraleinheit 200 mit elektrischer Energie versorgt werden. Ein an einer abgeschrägten Oberseite des Chassis 201 angebrachter Gassensor 206 erlaubt eine Messung einer Konzentration eines Gases in der Umgebung der Zentraleinheit, in der sich auch eine nicht dargestellte Bedienperson befinden kann. Das Gas kann insbesondere das chemische Dekontaminationsmittel sein. Mittels des Gassensors 206 kann eine Gefährdung der Bedienperson verhindert werden, sofern im Falle einer Detektion einer gesundheitsgefährdenden Gaskonzentration eine entsprechende Alarmmeldung ausgegeben wird.

Die Zentraleinheit 200 weist ferner eine Signalsäule 207 auf, welche den Betriebszustand der Zentraleinheit 200 visuell anzeigen kann. Außerdem ist eine Benutzerschnittstelle 215 vorgesehen. Gemäß dem hier dargestellten Ausführungsbeispiel ist die Benutzerschnittstelle ein berührungsempfindlicher Bildschirm 215. Mittels des Bildschirms 215 können Betriebszustände der Zentraleinheit 200 oder die Betriebszustände der einzelnen Komponenten der Zentraleinheit einer Bedienperson angezeigt werden. Ferner können über den berührungsempfindlichen Bildschirm 215 von einer Bedienperson Benutzereingaben, welche an die Steuerungseinheit 110 weitergeleitet werden, eingegeben werden.

An der Seitenwand des Chassis 201 befindet sich eine Öffnung 208 über welche in der Zentraleinheit 200 erzeugte Wärme entweichen kann. Dadurch kann eine unerwünschte Überhitzung der Zentraleinheit 200 vermieden werden. Ferner befindet sich an der Seitenwand ein Fluideingang 222 und ein Fluidausgang 228. Mittels des Fluideingangs 222 kann Raumluft von dem zu dekontaminierenden Raum in die Zentraleinheit 200 zurückgeführt werden. Mittels des Fluidausgangs 228 kann das Fluid bzw. das Abgabefluid einer in dem zu dekontaminierenden Raum befindlichen Abgabeeinheit zugeführt werden. Ferner sind Steueranschlüsse 225 für die Abgabeeinheit sowie Anschlüsse 224 für eine externe Generatoreinheit vorgesehen, mittels welcher ebenfalls das chemische Dekontaminationsmittel erzeugt und der Zentraleinheit zugeführt werden kann.

Die in diesem Dokument dargestellte Zentraleinheit 100, 200 wurde speziell für die Begasung und die Dekontamination von einzelnen oder mehreren Räumen entwickelt. Über die Zentraleinheit können eine oder mehrere Abgabeeinheiten wie beispielsweise Düseneinheiten oder Raumbegasungsinstallationen parallel oder seriell angesteuert und versorgt werden. Die Zentraleinheit ist bei der Verwendung einer externen Generatoreinheit somit eine Schnittstellenanlage zwischen dieser externen Generatoreinheit und dem zu dekontaminierenden Raum wie beispielseise einer Begasungskammer bzw. einem Begasungssystem. Der große Vorteil dabei liegt darin, dass größere Luftmengen mit unterschiedlichen Luftzuständen und Luftdrücken bewegt werden können, dass evaluierte Begasungszustände pro Raum oder pro Kammer gespeichert und abgefahren werden können und dass die Begasung von der Generatoreinheit unabhängig erfolgen kann.

Die Zentraleinheit weist ein sauber verarbeitetes Schrankgehäuse aus Edelstahl auf, welches an die jeweiligen Einbaukomponenten und Funktionen, die von der Zentraleinheit erfüllt werden sollen, angepasst ist. Im Innenbereich der Zentraleinheit sind alle notwendigen Ventile, Ventilatoren, Bypassleitungen, die Generatoreinheit wie beispielse ein H2O2 Generator, ein Katalysator zum optionalen Abbau des Dekontaminationsmedium, eine Reihe von Fühlern und Sensoren, Absperrorgane und die gesamte Verrohrung eingebaut. Die Anschlüsse sind nach außen ausgeführt. Die Verbindungsleitungen zu dem zu dekontaminierenden Raum (Zuführungs- und Rückführungsleitung) sowie die Zuluft- und Abluftleitung können von außen an die Zentraleinheit angedockt werden. Die Zentraleinheit ist mit einer eigenen Regelung, Steuerung und einer speziellen Software ausgestattet. Die Abmessung und die technischen Daten richten sich nach dem jeweiligen Einsatzfall und Ausstattung. Die Zentraleinheit ist als Modulsystem konzipiert und kann mit verschiedensten Komponenten bestückt werden und somit mehrere Funktionen erfüllen.

In der Zentraleinheit ist eine Speicher programmierbare Steuerung wie beispielsweise die Siemens SPS Serie S7-300 sowie ein berührungsempfindlicher Bildschirm wie beispielsweise das Siemens Touch Panel MP377 15" eingebaut. Die gesamte Steuerung und Regelung wird von dieser SPS übernommen.

Am Touch Panel ist die gesamte Visualisierung, die für die Bedienung der Anlage notwendig ist, installiert. Die Programmsicherung kann mittels einer Memory Card erfolgen. Die Visualisierung und die Passwortverwaltung kann mit einer Software für eine Prozessvisualisierung wie beispielsweise der sog. WinCC der Firma Siemens umgesetzt werden. Ferner kann ein Drucker angeschlossen werden oder auch in der Zentraleinheit integriert sein, mit dem man prozessrelevante Informationen wie beispielsweise Chargenprotokolle und/oder ein Dekontaminationsprotokolle ausdrucken kann.

Bezugszeichenliste:
- 100: Zentraleinheit
- 110: Steuerungseinheit
- 120: Fluidleitung
- 122: Fluideingang
- 122a: weiterer Fluideingang
- 122b: weiterer Fluideingang
- 128: Anschluss / Fluidausgang
- 128a: weiterer Anschluss /weiterer Fluidausgang
- 128b: weiterer Anschluss /weiterer Fluidausgang
- 130: Ventilatoreinheit
- 132: Zuluftleitung
- 134: Abluftleitung
- 140: Generatoreinheit / H2O2 Generator
- 150: erste Bypassleitung
- 152: Verteileinheit
- 154: Abbaueinheit
- 156: Filtereinheit
- 160: zweite Bypassleitung
- 161: Entfeuchtungseinheit
- 163: Erhitzereinheit
- 165: Kühlungseinheit
- 167: Befeuchtungseinheit
- 169: Verteileinheit
- 169a: Messeinrichtung für Volumenstrom
- 170: Hilfseinheit
- 180: Abgabeeinheit / Düseneinheit
- 181: Verbindungsleitung
- 185: Lüftungssystem
- 190: zu dekontaminierender Raum

- 200: Zentraleinheit
- 201: Chassis / Gehäuse
- 202: Rollen
- 203: Hauptschalter
- 204: Notausschalter
- 205: Steckdose
- 206: Gassensor
- 207: Signalsäule
- 208: Öffnung für Wärmeabfuhr
- 215: Benutzerschnittstelle / berührungsempfindlicher Bildschirm
- 222: Fluideingang (Rücklauf)
- 225: Steueranschlüsse für Abgabeeinheit
- 224: Anschlüsse für externe Generatoreinheit
- 228: Fluidausgang (Vorlauf)

## Patentansprüche

1. Zentraleinheit für ein Dekontaminationssystem zum chemischen Dekontaminieren von Räumen, die Zentraleinheit (100, 200) aufweisend
• einen Anschluss (128) zum Anschließen einer Abgabeeinheit (180), welche zum Abgeben eines chemischen Dekontaminationsmittels in einen zu dekontaminierenden Raum (190) eingerichtet ist,
• eine Steuerungseinheit (110) zum Steuern einer Zufuhr des chemischen Dekontaminationsmittels zu der Abgabeeinheit (180),
• eine mit der Steuerungseinheit (110) gekoppelte Benutzerschnittstelle (215) zum Eingeben von zumindest einer Information an die Steuerungseinheit (110),
- wobei die Information mit einem benutzerdefinierten Dekontaminationsprozess verknüpft ist und
- wobei die Steuerungseinheit (110) derart eingerichtet ist, dass basierend auf der Information der entsprechende benutzerdefinierte Dekontaminationsprozess von der Zentraleinheit (110) durchführbar ist, und
• eine Fluidleitung (120), welche
ausgangsseitig mit dem Anschluss (128) gekoppelt ist und welche derart eingerichtet ist, dass
ein Fluid, welches das chemische Dekontaminationsmittel aufweist, zu dem Anschluss (128) transferierbar ist, **dadurch gekennzeichnet, daß**
die Fluidleitung (120) eine Unterbrechung und durch die Unterbrechung erzeugte Enden aufweist, an welchen eine in Bezug zu der Zentraleinheit externe Generatoreinheit zum Erzeugen des chemischen Dekontaminationsmittels anschließbar ist, oder
die Fluidleitung (120) einen Teilabschnitt aufweist, zu dem eine in Bezug zu der Zentraleinheit externe Generatoreinheit zum Erzeugen des chemischen Dekontaminationsmittels parallel schaltbar ist.

2. Zentraleinheit nach dem vorangehenden Anspruch, ferner aufweisend
• einen Fluideingang (122), welcher eingangsseitig mit der Fluidleitung (120) gekoppelt ist und welcher zum Einspeisen von einem Fluid aus dem zu dekontaminierenden Raum in die Fluidleitung (120) eingerichtet ist.

3. Zentraleinheit nach einem der Ansprüche 1 bis 2, ferner aufweisend
• eine Ventilatoreinheit (130), welche in der Fluidleitung (120) angeordnet ist.

4. Zentraleinheit nach einem der Ansprüche 1 bis 3, ferner aufweisend
• eine interne Generatoreinheit (140) zum Erzeugen des chemischen Dekontaminationsmittels, wobei die interne Generatoreinheit (140) in der Fluidleitung (120) angeordnet ist.

5. Zentraleinheit nach einem der Ansprüche 1 bis 4, ferner aufweisend
• eine Abbaueinheit (154) zum zumindest teilweisen Abbauen des chemischen Dekontaminationsmittels, wobei die Abbaueinheit in der Fluidleitung (120) angeordnet ist.

6. Zentraleinheit nach einem der Ansprüche 1 bis 5, ferner aufweisend
• eine Filtereinheit (156), welche in der Fluidleitung (120) angeordnet ist.

7. Zentraleinheit nach einem der Ansprüche 1 bis 6, ferner aufweisend
• eine Feuchtigkeitsbeeinflussungseinheit (161, 167), welche in der Fluidleitung (120) angeordnet ist.

8. Zentraleinheit nach einem der Ansprüche 1 bis 7, ferner aufweisend
• eine Temperiereinheit (163, 165), welche in der Fluidleitung (120) angeordnet ist.

9. Zentraleinheit nach einem der Ansprüche 1 bis 8, ferner aufweisend
• eine Messeinrichtung (169a) zum Messen des Volumenstroms des in der Fluidleitung (120) geförderten Fluids.

10. Zentraleinheit nach einem der vorangehenden Ansprüche, ferner aufweisend
• einen weiteren Anschluss (128a, 128b) zum Anschließen einer weiteren Abgabeeinheit, welche zum Abgeben eines chemischen Dekontaminationsmittels in einen weiteren zu dekontaminierenden Raum eingerichtet ist,
wobei die Steuerungseinheit (110) ferner eingerichtet ist zum Steuern einer Zufuhr des chemischen Dekontaminationsmittels zu der weiteren Abgabeeinheit.

11. Zentraleinheit nach dem vorangehenden Anspruch, ferner aufweisend
• eine erste Verteileinheit (169), welche einen Eingang, einen ersten Ausgang und einen zweiten Ausgang aufweist,
- wobei der Eingang mit einem Ausgang der Fluidleitung (120) gekoppelt ist,
- wobei der erste Ausgang mit dem Anschluss (128) zum Anschließen der Abgabeeinheit (180) gekoppelt ist und
- wobei der zweite Ausgang mit dem weiteren Anschluss (128a) zum Anschließen der weiteren Abgabeeinheit gekoppelt ist.

12. Zentraleinheit nach einem der beiden vorangehenden Ansprüche, ferner aufweisend
• eine zweite Verteileinheit (152), welche einen ersten Eingang, einen zweiten Eingang und einen Ausgang aufweist,
- wobei der erste Eingang dem zu dekontaminierenden Raum (190) zugeordnet ist,
- wobei der zweite Eingang dem weiteren zu dekontaminierenden Raum zugeordnet ist und
- wobei der Ausgang mit einem Eingang der Fluidleitung (120) gekoppelt ist.

13. Dekontaminationssystem zum chemischen Dekontaminieren von Räumen, das Dekontaminationssystem aufweisend
• eine Zentraleinheit (100) nach einem der vorangehenden Ansprüche und
• eine Abgabeeinheit (180) zum Abgeben eines chemischen Dekontaminationsmittels in den zu dekontaminierenden Raum (190), wobei
die Zentraleinheit (100) und die Abgabeeinheit (180) über eine Verbindungsleitung (181) miteinander gekoppelt sind.

14. Dekontaminationssystem nach dem vorangehenden Anspruch, wobei die Abgabeeinheit eine mobile Abgabeeinheit (180) oder eine stationäre Abgabeeinheit (180) ist.

15. Verfahren zum chemischen Dekontaminieren von Räumen, das Verfahren aufweisend
• Eingeben von zumindest einer Information an eine Steuerungseinheit (110) einer Zentraleinheit (100) mittels einer mit der Steuerungseinheit (110) gekoppelten Benutzerschnittstelle (215) der Zentraleinheit (100), wobei die Information mit einem benutzerdefinierten Dekontaminationsprozess verknüpft ist,
• Erzeugen des chemischen Dekontaminationsmittels mittels einer in Bezug auf die Zentraleinheit externen Generatoreinheit,
• Transferieren des chemischen Dekontaminationsmittels zu einem Anschluss (128) der Zentraleinheit (100) mittels einer Fluidleitung (120), an welcher die externe Generatoreinheit angeschlossen ist,
• Abgeben eines chemischen Dekontaminationsmittels in einen zu dekontaminierenden Raum (190) mittels einer Abgabeeinheit (180), welche über den Anschluss (128) mit der Zentraleinheit (100) gekoppelt ist, und
• Steuern einer Zufuhr des chemischen Dekontaminationsmittels zu der Abgabeeinheit (180) mittels der Steuerungseinheit (110),
wobei die Steuerungseinheit (110) derart eingerichtet ist, dass basierend auf der Information der entsprechende benutzerdefinierte Dekontaminationsprozess von der Zentraleinheit (110) durchgeführt wird und wobei die externe Generatoreinheit an durch eine Unterbrechung der Fluidleitung (120) erzeugte Enden angeschlossen ist oder
wobei die externe Generatoreinheit parallel zu einem Teilabschnitt der Fluidleitung (120) geschaltet ist.

## Claims

1. A central unit for a decontamination system for chemically decontaminating rooms, the central unit (100, 200) comprising
- a connector (128) for connecting a delivery unit (180) arranged for delivering a chemical decontamination agent to a room (190) being to be decontaminated,
- a control unit (110) for controlling a supply of the chemical decontamination agent to the delivery unit (180),
- an user interface (215) coupled to the control unit (110) for inputting at least one information to the control unit (110),
- wherein the information is linked with a user defined decontamination process and
- wherein the control unit (110) is arranged so that a respective user defined decontamination process is performable by the central unit (110) based on the information and
- a fluid conduit (120) which is coupled to the connector (128) at an output side and which is arranged so that
a fluid comprising the chemical decontamination agent is transferable to the connector (128), **characterized in that**
the fluid conduit (120) comprises an intermission and ends generated by the intermission, at which ends a generator unit for generating the chemical decontamination agent is connectable, which generator unit is external with respect to the central unit, or
the fluid conduit (120) comprises a section to which a generator unit for generating the chemical decontamination agent is connectable in parallel, which generator unit is external with respect to the central unit.

2. The central unit according to the preceding claim, further comprising
- a fluid inlet (122) which is coupled to the fluid conduit (120) at an input side and which is arranged for introducing a fluid from the room to be decontaminated into the fluid conduit (120).

3. The central unit according to any one of the claims 1 to 2, further comprising
- a fan unit (130) disposed in the fluid conduit (120).

4. The central unit according to any one of the claims 1 to 3, further comprising
- an internal generator unit (140) for generating the chemical decontamination agent, wherein the internal generator unit (140) is disposed in the fluid conduit (120).

5. The central unit according to any one of the claims 1 to 4, further comprising
- a dissipation unit (154) for at least partially dissipating the chemical decontamination agent, wherein the dissipation unit is disposed in the fluid conduit (120).

6. The central unit according to any one of the claims 1 to 5, further comprising
- a filter unit (156) disposed in the fluid conduit (120).

7. The central unit according to any one of the claims 1 to 6, further comprising
- a humidity influencing unit (161, 167) disposed in the fluid conduit (120).

8. The central unit according to any one of the claims 1 to 7, further comprising a tempering unit (163, 165) disposed in the fluid conduit (120).

9. The central unit according to any one of the claims 1 to 8, further comprising
- a measuring unit (169 a) for measuring the volume flux of a flux conveyed in the fluid conduit (120).

10. The central unit according to any one of the preceding claims, further comprising
- a further connector (128 a, 128b) for connecting of a further delivery unit arranged for delivering a chemical decontamination agent to a further room being to be decontaminated,
wherein the control unit (110) is further arranged for controlling a supply of the chemical decontamination agent to the further delivery unit.

11. The central unit according to the preceding claim, further comprising
- a first distribution unit (169) comprising an inlet, a first outlet and a second outlet,
- wherein the inlet is coupled to an outlet of the fluid conduit (120),
- wherein the first outlet is coupled to the connector (128) for connecting the delivery unit (180) and
- wherein the second outlet is coupled to the further connector (128 a) for connecting the further delivery unit.

12. The central unit according to any one of the two preceding claims, further comprising
- a second distribution unit (152) comprising a first inlet, a second inlet and an outlet,
- wherein the first inlet is associated to the room (190) being to be decontaminated,
- wherein the second inlet is associated with the further room being to be decontaminated and
- wherein the outlet is coupled to an inlet of the fluid conduit (120).

13. A decontamination system for chemically decontaminating rooms, the decontamination system comprising
- a central unit (100) according to any one of the preceding claims and
- a delivery unit (180) for delivering a chemical decontamination agent to the room (190) being to be decontaminated, wherein the central unit (100) and the delivery unit (180) are coupled together via a connecting conduit (181).

14. The decontamination system according to the preceding claim, wherein the delivery unit is a mobile delivery unit (180) or a stationery delivery unit (180).

15. A method for chemically decontaminating rooms, the method comprising
- inputting at least one information to a control unit (110) of a central unit (100) by a user interface (215) of the central unit (100), which is coupled to the control unit (110), wherein the information is linked with a user defined decontamination process,
- generating the chemical decontamination agent by a generator unit which is external with respect to the central unit,
- transferring the chemical decontamination agent to a connector (128) of the central unit (100) by a fluid conduit (120), at which the external generator unit is connected,
- delivering a chemical decontamination agent in a room (190) being to be decontaminated by a delivery unit (180) coupled to the central unit (100) via a the connector (128) and
- controlling a supply of the chemical decontamination agent to the delivery unit (180) by the control unit (110),
wherein the control unit (110) is arranged so that, based on the information, the respective user defined decontamination process is performed by the central unit (110) and
wherein the external generator unit is connected to ends which are generated by an intermission of the fluid conduit (120) or
wherein the external generator unit is connected parallel to a section of the fluid conduit 120.

## Revendications

1. Unité centrale destinée à un système de décontamination servant à décontaminer par voie chimique des pièces, ladite unité centrale (100, 200) présentant :
- un dispositif de raccordement (128) servant à raccorder une unité de distribution (180), laquelle est mise en place pour distribuer un agent de décontamination chimique dans une pièce (190) devant être décontaminée,
- une unité de commande (110) servant à commander une amenée de l'agent chimique de décontamination à l'unité de distribution (180),
- une interface utilisateur (215) couplée à l'unité de commande (110) et servant à enregistrer au moins une information à l'attention de l'unité de commande (110),
- sachant que l'information est associée à un processus de décontamination défini par l'utilisateur et
- sachant que l'unité de commande (110) est mise en place de telle manière que le processus de décontamination correspondant défini par l'utilisateur peut être exécuté, sur la base de l'information, par l'unité centrale (110),
et
- une conduite de fluide (120), qui
est couplée côté sortie au dispositif de raccordement (128) et qui est mise en place de telle manière
qu'un fluide présentant l'agent chimique de décontamination peut être transféré en direction du dispositif de raccordement (128), **caractérisée en ce**
**que** la conduite de fluide (120) présente une interruption et des extrémités produites par l'interruption, au niveau desquelles peut être raccordée une unité génératrice externe par rapport à l'unité centrale servant à produire l'agent chimique de décontamination, ou
**que** la conduite de fluide (120) présente une section partielle, par rapport à laquelle une unité génératrice externe par rapport à l'unité centrale servant à produire l'agent chimique de décontamination peut être montée en parallèle.

2. Unité centrale selon la revendication précédente, présentant en outre
- une entrée pour fluide (122), qui est couplée côté entrée à la conduite de fluide (120) et qui est mise en place pour injecter un fluide dans la conduite de fluide (120) depuis la pièce devant être décontaminée.

3. Unité centrale selon l'une quelconque des revendications 1 à 2, présentant en outre
- une unité de ventilation (130) qui est disposée dans la conduite de fluide (120).

4. Unité centrale selon l'une quelconque des revendications 1 à 3, présentant en outre
- une unité génératrice interne (140) servant à produire l'agent chimique de décontamination, sachant que l'unité génératrice interne (140) est disposée dans la conduite de fluide (120).

5. Unité centrale selon l'une quelconque des revendications 1 à 4, présentant en outre
- une unité d'élimination (154) servant à éliminer au moins en partie l'agent chimique de décontamination, sachant que l'unité d'élimination est disposée dans la conduite de fluide (120).

6. Unité centrale selon l'une quelconque des revendications 1 à 5, présentant en outre
- une unité de filtration (156), qui est disposée dans la conduite de fluide (120).

7. Unité centrale selon l'une quelconque des revendications 1 à 6, présentant en outre
- une unité influençant l'humidité (161, 167) qui est disposée dans la conduite de fluide (120).

8. Unité centrale selon l'une quelconque des revendications 1 à 7, présentant en outre
- une unité de régulation thermique (163, 165) qui est disposée dans la conduite de fluide (120).

9. Unité centrale selon l'une quelconque des revendications 1 à 8, présentant en outre
- un système de mesure (169a) servant à mesurer le débit volumique du fluide acheminé dans la conduite de fluide (120).

10. Unité centrale selon l'une quelconque des revendications précédentes, présentant en outre
- un autre dispositif de raccordement (128a, 128b) servant à raccorder une autre unité de distribution, laquelle est mise en place pour distribuer un agent chimique de décontamination dans une autre pièce devant être décontaminée,
sachant que l'unité de commande (110) est en outre mise en place pour commander une amenée d'agent chimique de décontamination en direction de l'autre unité de distribution.

11. Unité centrale selon la revendication précédente, présentant en outre
- une première unité de répartition (169) qui présente une entrée, une première sortie et une deuxième sortie,
- sachant que l'entrée est couplée à une sortie de la conduite de fluide (120),
- sachant que la première sortie est couplée au dispositif de raccordement (128) servant à raccorder l'unité de distribution (180), et
- sachant que la deuxième sortie est couplée à l'autre dispositif de raccordement (128a) servant à raccorder l'autre unité de distribution.

12. Unité centrale selon l'une quelconque des deux revendications précédentes, présentant en outre
- une deuxième unité de répartition (152) qui présente une première entrée, une deuxième entrée et une sortie,
- sachant que la première entrée est associée à la pièce (190) devant être décontaminée,
- sachant que la deuxième entrée est associée à l'autre pièce devant être décontaminée, et
- sachant que la sortie est couplée à une entrée de la conduite de fluide (120).

13. Système de décontamination servant à décontaminer par voie chimique des pièces, ledit système de décontamination présentant
- une unité centrale (100) selon l'une quelconque des revendications précédentes et
- une unité de distribution (180) servant à distribuer un agent chimique de décontamination dans la pièce (190) devant être décontaminée, sachant que l'unité centrale (100) et l'unité de distribution (180) sont couplées l'une à l'autre par l'intermédiaire d'une conduite de connexion (181).

14. Système de décontamination selon la revendication précédente, sachant que l'unité de distribution est une unité de distribution (180) mobile ou une unité de distribution (180) immobile.

15. Procédé servant à décontaminer par voie chimique des pièces, ledit procédé présentant des étapes consistant à :
- enregistrer au moins une information à l'attention d'une unité de commande (110) d'une unité centrale (100) au moyen d'une interface utilisateur (215), couplée à l'unité de commande (110), de l'unité centrale (100), sachant que l'information est associée à un processus de décontamination défini par l'utilisateur ;
- produire l'agent chimique de décontamination au moyen d'une unité génératrice externe par rapport à l'unité centrale ;
- transférer l'agent chimique de décontamination en direction d'un dispositif de raccordement (128) de l'unité centrale (100) au moyen d'une conduite de fluide (120), à laquelle est raccordée l'unité génératrice externe ;
- distribuer un agent chimique de décontamination dans une pièce (190) devant être décontaminée au moyen d'une unité de distribution (180) qui est couplée à l'unité centrale (100) par l'intermédiaire du dispositif de raccordement (128) ; et
- commander une amenée de l'agent chimique de distribution à une unité de distribution (180) au moyen de l'unité de commande (110),
sachant que l'unité de commande (110) est mise en place de telle manière que le processus de décontamination correspondant défini par l'utilisateur est exécuté, sur la base de l'information, par l'unité centrale (110), et
sachant que l'unité génératrice externe est raccordée à des extrémités produites par une interruption de la conduite de fluide (120), ou
sachant que l'unité génératrice externe est montée en parallèle par rapport à la section partielle de la conduite de fluide (120).
